# EUROPÄISCHE PATENTSCHRIFT

(11) **EP 0 491 362 B1**
(45) Veröffentlichungstag und Bekanntmachung des Hinweises auf die Patenterteilung: **13.03.1996**
(21) Anmeldenummer: 91121625.7
(22) Anmeldetag: 17.12.1991
(51) Int. Cl.: G01N 33/68, G01N 33/543

(54) **Verwendung von Peptidpaaren mit extrem hoher spezifischer Affinität zueinander im Bereich der in vitro Diagnostik**
Use of peptide pairs with extremely high mutual specific affinity in the domain of in-vitro diagnostics
Utilisation de paires de peptides ayant une affinité spécifique réciproque extrêmement élevée dans le domaine du diagnostic in vitro

(30) Priorität: 19.12.1990 DE 4040669
(43) Veröffentlichungstag der Anmeldung: 24.06.1992
(73) Patentinhaber: BEHRINGWERKE Aktiengesellschaft, D-35001 Marburg (DE)
(72) Erfinder: Bosslet, Klaus, Dr., W-3550 Marburg (DE); Hermentin, Peter, Dr., W-3550 Marburg (DE); Sedlacek, Hans Harald, Dr., W-3550 Marburg (DE); Auerbach, Bernhard, Dr., W-3550 Marburg (DE); Pfleiderer, Peter, Dr., W-3550 Marburg (DE); Müller, Rolf, Prof. Dr., W-3550 Marburg (DE)
(74) Vertreter: Aulmich, Gerhard, Dr.

(56) Entgegenhaltungen:
- US-A- 4 228 237
- SCIENCE, Band 245, 11. August 1989, Lancaster, PA (US); E.K. O'SHEA et al., Seiten 646-648/
- NATURE, Band 341, 21. September 1989, London (GB); M. NEUBERG et al., Seiten 243-245/

## Beschreibung

Die Erfindung betrifft die Verwendung bestimmter Peptide mit extrem hoher Affinität zu anderen Peptiden (Heterodimerbildung) und niedriger Affinität zu sich selbst (Homodimerbildung) im Bereich der in vitro Diagnostik.

Es sind zahlreiche Beispiele dafür bekannt, bei denen zwei (Bio)Moleküle durch stark begünstigte Interaktionen hohe Bindungsaffinitäten zueinander ausbilden können. Als ein Beispiel sei hier auf die spezifischen, gerichteten Wechselwirkungen zwischen Antikörpern und Antigenen hingewiesen auf deren Basis immunologische Prozesse ablaufen und deren praktische Nutzung die in-vitro Diagnostik sehr bereichert haben. Die Verwendung von solchen spezifischen Bindungspartnern (z.B. monoklonale Antikörper, Lektine, Rezeptoren u.a.) haben es ermöglicht, hochspezifisch diagnostisch relevante Antigene zu erkennen. Neben der Frage der Spezifität ist die der Sensitivität für die Diagnostik zur Detektion der Biokomplexe von gleichrangiger Bedeutung.

Aus verschiedenen Gründen hat es sich als zweckmäßig erwiesen bei diagnostischen Verfahren, bei denen ein spezifischer Bindungspartner eines Analyten z. B. kovalent oder adsorptiv an eine Festphase gebunden ist, diesen spezifischen Bindungspartner nicht direkt, sondern über ein weiteres spezifisches Bindungspaar, das nicht mit dem Analyten reagiert, an die Festphase zu koppeln (im weiteren "universelle Festphase" genannt).

Ferner hat es sich als zweckmäßig erwiesen, daß es bei Verfahren, bei denen der Einsatz der spezifischen Biomoleküle durch ihre Stabilität und Potenz limitiert ist, sinnvoll sein kann, auch bei bestimmten Reagenzien, z. B. Enzymkonjugaten, eine entsprechende, indirekte Methode zu verwenden, d. h. daß der spezifische Bindungspartner über ein weiteres spezifisches Bindungspaar, das nicht mit dem Analyten oder dem ersten spezifischen Bindungspartner reagiert, mit der signalgebenden Komponente verknüpft ist.

Dadurch kann z. B. ein "universelles Konjugat" für verschiedene Analyten eingesetzt werden.

Zur Realisierung einer "universellen Festphase" werden z. B. Maus-Immunglobulin-spezifische Reagenzien wie Anti-Maus-Antikörper bzw. antikörperspezifische Reagenzien wie Protein A verwendet, die nach herkömmlichen Verfahren auf die Festphase aufgebracht werden (Practice and Theory of Enzyme Immunoassays, P. Tijssen, Elsevier, 1988). Die Attraktivität dieses Konzeptes liegt u. a. darin begründet, daß eine so hergestellte Festphase in der Lage ist, jeden spezifischen Maus-Antikörper gerichtet und reproduzierbar aufgrund von immunologischer Reaktion anzubinden. Die Nutzung einer solchermaßen hergestellten Festphase ist grundsätzlich als klassische stationäre Festphase ("Fertigreagenz") oder aber auch in homogener Phase während der immunologischen Reaktion möglich.

Immunchemische Nachweisverfahren und im speziellen heterogene immunchemische Nachweisverfahren sind dem Fachmann an sich bekannt. Als Nachteile der bekannten "universellen Festphasen" seien hier die Begrenzung ausschließlich auf Antikörperbeschichtungen genannt, wie auch die Limitation auf bestimmte Testkonstellationen (keine vollmonoklonalen ELISA-Versionen, keine Antigenfestphasen).

Weiterhin ist bekannt, daß Proben mit merklichen HAMA-Titern (HAMA = Humane Anti-Maus-Antikörper) nach in vivo-Applikation monoklonaler Antikörper am Menschen solche Testaufbauten, und solche in homogener Phase durchgeführt im besonderen Maße, ganz empfindlich stören können und man somit zu falschen diagnostischen Aussagen kommen kann.

Ein weiteres System zur Realisierung besagter Konzepte ist das mit Biotin/Avidin (z. B. US 4,228,237). Es besitzt sicherlich eine Sonderstellung bezüglich der beobachtbaren hohen resultierenden Bindungsstärken. Die vielfältige Nutzung dieses Systems ist in einem Übersichtsartikel beschrieben (Methods in Enzymology, Vol. 184 (1990), Avidin-Biotin Technology, Ed. Meir Wilchek; E.A. Bayer).

Aus der Natur des Avidinmoleküls erwachsen aber auch die Nachteile dieser Systemkomponente innerhalb der in vitro Diagnostik. So besitzt das Glykoprotein Avidin einen überaus hohen isoelektrischen Punkt (pI) und bildet daher bei neutralem pH-Wert, also unter physiologischen Bedingungen, als stark positiv geladenes Molekül starke unspezifische Wechselwirkungen mit negativ geladenen Molekülen wie z.B. Nukleinsäuren, Phospholipiden, etc. bzw. auch mit geladenenen Oberflächen aus. Es ist weiter bekannt, daß aufgrund von Mikroheterogenitäten der Glykoproteine keine definierten und streng reproduzierbaren Produkte hergestellt werden können.Der Kohlehydratanteil im Molekül trägt desweiteren zu unerwünschten Nebenreaktionen mit anderen biologischen Molekülen (z.B. "endogenes Lektin") bei, die zu deutlichen Sensitivitätseinbußen führen können. Ein Hinweis auf unerwünschte Bindung von Avidin zu endogenem Lektin ist in dem schon genannten Literaturzitat enthalten.

Diese Nachteile können zum Großteil durch Verwendung des Analogs Streptavidin aus dem Bakterium Streptomyces avidinii umgangen werden. Dieses hat nahezu gleichhohe Affinitäten zu Biotin. Es besitzt einen pI-Wert um den Neutralpunkt und trägt ferner auch keine Kohlenhydrate im Molekül. Dennoch ergeben sich auch beim Streptavidin/Biotin-System grundsätzliche Probleme, die zu empfindlichen Störungen und bei Einsatz innerhalb der in vitro Diagnostik eventuell zu unzuverlässigen Ergebnissen führen können. Es ist bekannt, daß das Vitamin Biotin von allen lebenden Zellen benötigt wird und somit ubiquitär in allen Geweben und Körperflüssigkeiten ist. So beträgt zum Beispiel die Konzentration an Biotin im Humanserum ungefähr 10 ng/ml. Daraus folgt bei allen diagnostischen Anwendungen eine Kompetition des Reagenz mit dem Probenmaterial, dessen Biotingehalt in Abhängigkeit vom physiologischen Zustand des Individuums und seinen Ernährungsgewohnheiten (z.B. Einnahme von Multivitaminpräparaten) verschieden stark schwanken kann.

Ein weiteres gravierendes Störpotential resultiert aus der in vivo Anwendung des Streptavidin/Biotin-Systems (G. Paganelli et al., Radiolocalisation of tumour pretargeted by biotinylated monoclonal antibody; Advances in the applications of monoclonal antibodies in clinical oncology; abstracts). Dabei geht man so vor, daß ein biotinylierter Antikörper appliziert wird und man circa 3-5 Tage später Indium-markiertes Streptavidin folgen läßt. Es ist bekannt, daß sowohl Avidin wie auch Streptavidin stark immunogene Substanzen sind und daher nach Applikation zu einer Human-Antwort führen. Man spricht hierbei von sogenannten HASAs, also humanen-anti-Streptavidin-Antikörpern, die in Testaufbauten innerhalb der Serumdiagnostik zu großen Problemen führen können.

Die vorliegende Erfindung hat sich die Aufgabe gestellt ein hochaffines System zur Nutzung innerhalb der in vitro Diagnostik zur Verfügung zu stellen, das die oben genannten Nachteile nicht aufweist. Dabei sollten die Einzelkomponenten zudem mit etablierten Verfahrensweisen leicht, reproduzierbar und kontaminationsfrei zugänglich sein.

Das erfindungsgemäße Verfahren beruht auf den überaus starken Wechselwirkungen von Peptidabschnitten bestimmter Proteine, die in der Lage sind, DNA anzubinden und somit die Transkription zu modulieren vermögen. Es wurde gefunden, daß die Bindung z.B. des fos-Genproduktes an das jun-Genprodukt unter Bildung eines Heterodimers von außergewöhnlicher Stärke erfolgt. Diese Bindung zeigt ferner eine Resistenz gegen hohe Konzentrationen chaotroper Substanzen (z.B. Harnstoff), Detergenzien und Salz sowie gegen niedere bzw. sehr hohe pH-Werte. Vergleichbare Heterodimer-Bindungsstärken wurden mit synthetischen Peptiden, die die Aminosäurepositionen 285 bis 324, den sogenannten Leucin-Reißverschluß des c-jun Proteins, sowie die Positionen 162 bis 201, den Leucin-Reißverschluß des v-fos Proteins ausmachen, gemessen. Diese Peptide haben folgende Aminosäuresequenz:
c-jun-"Leucin-Reißverschlußpeptid":
RIARLEEKVKTLKAQNSELASTANMLREQVAQLKQKVMNY
v-fos-"Leucin-Reißverschlußpeptid":
LTDTLQAETDQLEDKKSALQTEIANLLKEKEKLEFILAAY

Folgender Buchstabenkode wurde benutzt:
A= Alanin, C= Cystein, D= Asparaginsäure, E= Glutaminsäure, G= Glycin, I= Isoleucin, K= Lysin, L= Leucin, M= Methionin, N= Asparagin, Q= Glutamin, R= Arginin, S= Serin, T= Threonin, V= Valin, Y= Tyrosin

Nach dem jetzigen Kenntnisstand sind dabei die Leucinreste, die regelmäßig in Abständen von 7 Aminosäuren angeordnet sind (in den vorstehenden Formeln unterstrichen) wirksam. Das c-jun-"Leucin-Reißverschlußpeptid" zeigt im Gegensatz zum c-jun Genprodukt keine Homodimerisierung. Das v-fos-"Leucin-Reißverschlußpeptid" zeigt ähnlich wie das intakte v-fos Genprodukt keine starke Tendenz zur Homodimerisierung, bildet aber - wie oben bereits erwähnt - mit dem c-jun-"Leucin-Reißverschlußpeptid" sehr stabile heterodimere Komplexe (E.K. O'SHEA et al., Science 245, 646 - 648 (1989) und M. NEUBERG et al., Nature 341 243 - 245 (1989)).

"Leucin-Reißverschlußpeptide" im Sinne dieser Erfindung sind Peptide, die in der oben angegebenen Weise angeordnete Leucinreste enthalten, bevorzugt sind Peptide, die die c-jun und v-fos Sequenzen enthalten. Ganz besonders bevorzugt sind c-jun und v-fos.

Unerwarteterweise zeigten sich die "Leucin-Reißverschlußpeptide" bei ihrer erfindungsgemäßen Verwendung sowohl als Festphasenreagenzien, Fängerreagenzien, als auch als Nachweisreagenzien den konventionellen Reagenzien dieser Art (Protein A, Protein G, polyklonale oder monoklonale Antikörper) aufgrund ihrer extrem hohen Komplexstabilität überlegen.

Gegenstand der Erfindung ist die Verwendung von Peptidpaaren mit extrem hoher spezifischer Affinität zueinander im Bereich der in vitro Diagnostik, wobei diese Peptide "Leucin-Reißverschlußpeptide" sind.

Gegenstand der Erfindung ist ferner ein heterogenes Immunchemisches Verfahren zur Bestimmung eines Analyten, wobei der an die Festphase gebundene spezifische Bindungspartner über ein "Leucin-Reißverschluß" Peptidpaar an die Festphase gebunden ist.

Gegenstand der Erfindung ist auch eine universelle Festphase zur Verwendung in heterogenen immunchemischen Verfahren zur Bestimmung eines Analyten, wobei ein Peptid des "Leucin-Reißverschluß" Peptidpaares adsorptiv oder kovalent, direkt oder über einen Spacer an die Festphase gebunden ist.

Gegenstand der Erfindung ist auch ein universelles Reagenz zur Verwendung in immunchemischen Verfahren, wobei an den zum Nachweis dienenden spezifischen Bindungspartner mindestens ein Peptid des "Leucin-Reißverschluß" Peptidpaares gebunden ist und das zweite Peptid dieses Paares an eine signalgebende Komponente gebunden ist.

Durch das erfindungsgemäße Verfahren ist es auch möglich, an den zum Nachweis dienenden spezifischen Bindungspartner entweder direkt oder über ein Carriermolekül oder -partikel, mehr als ein Molekül des zweiten Peptids des "Leucin-Reißverschluß" Peptidpaares zu binden und so zu einer gezielt einstellbaren Signalerhöhung zu gelangen.

Gegenstand der Erfindung ist daher auch ein Verfahren zur Erhöhung des Signals vom Immunoassay, wobei an den spezifischen Bindungspartner direkt oder über ein Carriermolekül mehr als ein Molekül des zweiten Peptids des "Leucin-Reißverschluß" Peptidpaares gebunden ist.

Da sowohl das fos- und jun-"Reißverschlußpeptid" sowie die entsprechenden intakten Genprodukte aufgrund ihres hydrophilen Charakters gut wasserlöslich sind, können beide Peptide bzw. Genprodukte zur Kopplung an eine Festphase verwendet werden. Geeignete Festphasen sind dem Fachmann bekannt. Typische Beispiele für verwendbare Festphasen sind z. B. Röhrchen oder Formkörper aus einem Kunststoff, wie Polystyrol oder einem Polyamid wie z. B. Nylon, membranähnliche Strukturen oder magnetisierbare Partikel, z. B. paramagnetische Teilchen. Das jeweilige Peptid kann dabei direkt oder über einen Spacer, z. B. ein weiteres Peptid oder Protein oder ein kleineres Molekül, adsorptiv oder kovalent an die Festphase gebunden sein.

Das entsprechende komplementäre "Reißverschlußpeptid" kann z. B. an einen Antikörper oder ein Antigen nach dem Fachmann an sich bekannten Verfahren gebunden werden. Die Antikörper können mono- oder polyklonal sein. Antigene im Sinne der Erfindung können auch Haptene sein.

Weitere Beispiele für die erfindungsgemäße Verwendung sind kovalente Verknüpfungen obiger Peptide mit sonstigen Liganden sowie Enzymen, Rezeptoren, Zytokinen, Lymphokinen, katalytischen Antikörpern, Domänenantikörpern, Komplexonen und sonstigen Fusionsproteinen. Die kovalenten Verknüpfungen können sowohl über Sulfhydryl- und Aminogruppen wie auch über Kohlenhydratreste mittels geeigneter Linker erfolgen.

Da die Aminosäuresequenzen dieser "Reißverschlußpeptide" bekannt sind, ist bei dem heutigen Stand der Technik und der leichten Zugänglichkeit an Aminosäuren die Frage der Bioverfügbarkeit unproblematisch. Stand der Technik sind automatisierte Peptidsynthesizer, die innerhalb kurzer Zeit definierte, reproduzierbare und vor allem kontaminationsfreie Peptide zu liefern vermögen. Damit entfallen z. B. die zeitaufwendigen Isolierungsmethoden, deren Reproduzierbarkeit nicht zuletzt von der Qualität des aufgearbeiteten Materials abhängig ist. Erfindungswesentlich ist auch die Berücksichtigung einer gewünschten Funktionalisierung des Peptids im Syntheseschritt. Um z.B. eine spätere kovalente Verknüpfung des fos-Reißverschlußpeptids mit jedem gewünschten Trägerprotein über eine Sulfhydrylgruppe außerhalb der Leucin-Reißverschlußsequenz zu ermöglichen, wird am Aminoterminus z.B. ein Cystein über zwei Glycine mit dem fos-Leucin-Reißverschlußpeptid hinzusynthetisiert. Weitere ebenfalls geeignete Verlängerungspeptide könnten beliebig viele Aminosäuren und mehrere Cysteine enthalten. Die so erhaltene Cystein SH-Funktion kann dann z.B. zur Derivatisierung und/oder Kopplung an monoklonale Antikörper benutzt werden.

Dies kann z. B. so durchgeführt werden, daß man in einen Antikörper in an sich bekannter Weise Maleinimidofunktionen einführt, etwa durch Umsetzung mit N-(gamma-Maleinimidobutyryloxy)-succinimid, und das fos-Leucin-Reißverschlußpeptid über die terminale Cystein SH-Funktion an die Maleinimido-Doppelbindung addiert. Hier erfolgt die Kopplung über eine der Aminogruppen des MAk an die SH-Gruppe des Cysteins im fos-Peptid.

Alternativ kann eine Anknüpfung des fos- bzw. jun-Leucin-Reißverschlußpeptids an Antikörper auch über die Hinge-Region (Disulfidbrücken) des Antikörpers erfolgen, etwa dadurch, daß man das fos-Leucin-Reißverschlußpeptid über die terminale Cystein SH-Funktion in an sich bekannter Weise mit einem Bis-Maleinimid monofunktionalisiert und danach über die zweite eingeführte, noch unveränderte Maleinimido-Funktion an die Hinge-Thiol-Gruppen des z. B. mit Dithiothreit reduzierten Antikörpers addiert. Dieses zweite Verfahren hat den Vorteil, daß die Anknüpfung des fos- bzw. jun-Leucin-Reißverschlußpeptids an den Antikörper zielgerichtet abseits der Antigenbindungsstelle erfolgt.

In den Graphiken I-IV sind die grundsätzlichen Möglichkeiten eines breitgefächerten und sinnvollen Einsatzes der jun/fos-Wechselwirkung für die in vitro-Diagnostik beispielhaft schematisch dargestellt. Als ein wesentlicher Unterschied zu den schon oben genannten Technologien handelt es sich bei der jun/fos-Wechselwirkung um ein hochaffines System zweier niedermolekularer Substanzen. Aus den kleineren Molekulargewichten resultiert ein wesentlich höherer Flexibilisierungsgrad und damit die Realisierung von maßgeschneiderten Lösungen. Die Anbindung der Peptide an Biomoleküle ohne Verlust ihrer biologischen Aktivität ist aufgrund ihrer bescheidenen Raumbeanspruchung möglich. Die Limitation von Methoden, die durch massive sterische Hinderungen begrenzt ist, wird dadurch entschärft. Die kleinen Molekulargewichte ermöglichen desweiteren einfach zu synthetisierende Konstrukte, die als "Kupplerkomponente" z.B. der Streptavidin-Stöchiometrie von theoretisch vier Bindungszentren pro Molekül nicht nur überlegen sind, sondern auch frei gestaltet werden können. Als unmittelbare Konsequenz folgt daraus eine steuerbare Erhöhung der Sensitivität von diagnostischen Systemen.

### Erklärungen zu den Graphiken:

### Zu I) Festphase:

Hierbei kann die Bereitstellung der universellen Festphasen, die z.B. an Polystyrolgefäßen, Magnetpartikeln, Latexpartikeln etc. realisiert werden können, durch folgende Verfahrensweisen erfolgen:
- a): adsorptiv durch optimierte Beschichtungsverfahren
- b): kovalent durch chemische Verknüpfung mit einer funktionalisierten Festphase
- c): adsorptiv nach vorausgegangener kovalenter Verknüpfung eines Reißverschlußpeptids mit einem gut adsorbierbaren Carriermolekül
- d): kovalent durch Verknüpfung einer funktionalisierten Festphase mit einem Carriermolekül, an das chemisch ein Reißverschlußpeptid gebunden ist.

Die spezifische Festphase wird unter Nutzung der Peptid-Heterodimerbildung in einer Sekundärreaktion mit dem an den spezifischen Bindungspartner konjugierten komplementären Peptid hergestellt.

### Zu II) Konjugat:

An den spezifischen Bindungspartner wird nach den bekannten Kopplungsverfahren ein Reißverschlußpeptid mit den gewünschten Einbauraten konjugiert. Die Signalgebung erfolgt nach Sekundärreaktion mit einem, dem ersten komplementären Peptid, an das eine beliebige signalgebende Komponente (z.B. Enzym, Fluorogen, Luminogen, Radionuklid etc.) verknüpft wird.

### Zu III) Amplifikationssystem:

Die Amplifikation eines beliebigen Signals kann unter Nutzung der Reißverschlußpeptide auf folgende Weise erreicht werden:
- a): Ein zweiter spezifischer Bindungspartner der gegen einen das Antigen erkennenden, ersten spezifischen Bindungspartner gerichtet ist, wird entweder mehrfach mit einem Reißverschlußpeptid substituiert oder aus sterischen Gründen mit einem peptidtragenden Carriermolekül verknüpft.
- b): Der spezifische Bindungspartner wird mit dem gleichen Peptid wie die signalgebende Komponente substituiert. Die "Verkupplung" dieser beiden Komponenten erreicht man mit einem Carriermolekül, an dem das dem ersten komplementäre Peptid in der gewünschten Einbaurate gekoppelt ist.
- c): Die Amplifikation erfolgt unter Nutzung eines präformierten Komplexes zwischen der unter b) beschriebenen " Kupplerkomponente " und der mit Peptid versehenen signalgebenden Komponente.

### Zu IV) Artifizielles Kontrollserum/Standards:

Ein mit einem Reißverschlußpeptid verknüpfter spezifischer erster Bindungspartner erkennt das Antigen. In einer Sekundärreaktion bildet sich das Heterodimer mit einem dem ersten komplementären Peptid, das mit einer beliebigen Antigenstruktur (z.B. Peptid, Protein etc.) konjugiert ist und gegen die ein mit einer signalgebenden Komponente verknüpfter zweiter spezifischer Bindungspartner gerichtet ist.

Die Beispiele und die Ansprüche sind Bestandteil der Offenbarung der Erfindung.
Die Beispiele dienen der Erläuterung der Erfindung und schränkt sie in keiner Weise ein.

### Beispiel 1

### Schritt 1:

### Beschichtung von Mikrotitrationsplatten mit jun-Peptiden

150 µl einer 10 µg/ml jun-Peptid enthaltenden Lösung in 0,1 M Natriumcarbonat werden in jeweils 16 Näpfchen von Mikrotitrationsplatten der Firma Nunc, Roskilde, Dänemark gegeben. Die mit den Verdünnungen gefüllten Testplatten werden 18 Stunden bei 20°C belassen, dann werden die Lösungen in den Näpfchen abgesaugt und die Näpfchen 3 - 4 mal mit 300 µl einer Lösung von 10 g/l Rinderserumalbumin in phosphatgepufferter physiologischer Kochsalzlösung, (PBS, pH 7,4) durch Füllen und Absaugen gewaschen und die Testplatten anschließend über Kieselgel bei 20°C getrocknet.

### Schritt 2:

### Herstellung eines Maleimido-Antikörpers

Der zu konjugierende Antikörper (Konzentration 4 mg/ml in PBS; 2.5 ml) wird mit Lithiumboratpuffer (2.5 ml) vermischt (Herstellung s. u.). Zu der resultierenden Lösung mit einem pH von 7.5 werden unter Rühren 0.45 ml einer 13 mg/ml Lösung von GMBS (N-Gamma-Maleimidobutyryloxy-succinimid) in Dioxan (entsprechend einem 30fach molaren Überschuß vom GMBS gegenüber IgG) zugegeben. Nach einstündiger Inkubation bei Raumtemperatur wird der Reagenzüberschuß durch Gelfiltration an einer mit Phosphat gepufferten Kochsalzlösung (PBS), pH 7.2, äquilibrierten Sephadex G-25-Säule entfernt.

### Herstellung des Lithiumboratpuffers, pH 8.5:

1.24 g Borsäure werden in ein Gemisch aus Wasser (80 ml) und Dioxan (20 ml) gerührt. Unter Auflösung der Borsäure wird durch Zugabe von festem Lithiumhydroxid ein pH-Wert 8.5 eingestellt.

### Schritt 3:

### Kopplung des fos-Leucin-Reißverschlußpeptides an einen Maleimido-Antikörper

Der gemäß Schritt 2 hergestellte Maleimido-Antikörper (2 mg in 2 ml PBS) wird mit 1 equiv. fos-Leucin-Reißverschlußpeptid (64 µg in 100 µl PBS) 1 h bei Raumtemperatur inkubiert. Dann wird über eine AcA 34-Säule (Durchmesser 2,5 cm; Höhe 45 cm) mit einem Kochsalz/Natriumcitrat-Puffer, pH 7.3, (0.4 M Kochsalz und 0.04 M Natriumcitrat) gelfiltriert.

### Schritt 4:

### Herstellung eines Peroxidase-markierten Antikörpers sowie eines TMB-Substrats für die Detektion

Antikörper werden gemäß der Methode von KOEHLER und MILSTEIN zur Darstellung monoklonaler Antikörper erzeugt (Nature 256, 495, 1975), wobei verschiedene monoklonale Antikörper mit der gleichen Antigen-Spezifität mit der von STÄHLI et al. (J. of Immunological Methods 32, 297 - 304, 1980) beschriebenen Methode identifiziert wurden. Nach gelchromatographischer Reinigung und Dialyse gegen Phosphat-gepufferte Saline (PBS, pH 7,4) wird der die monoklonalen Antikörper-Fraktion enthaltende Pool (4 mg Protein/ml) anschließend mit N-gamma-Maleimidobutyryloxisuccinimid (GMBS), bezogen von der Fa. Calbiochem, umgesetzt, wie von TANAMORI et al. (J. Immunol. Meth. 62, 123 - 131, 1983) beschrieben.

Die so erhaltenen Lösungen der Antikörper-POD-Konjugate werden für die jeweilige Testanwendung endverdünnt. Als Konjugat-Verdünnungsmedium wird ein Puffer mit 0,1 M 2-Amino-2-(hydroxymethyl)-1,3-propandiol (TRIS), 0,5 % ^{R}Tween 20, 0,5 - 1 % Stabilisierungsprotein pH 7,4 verwendet.
Gemäß dem Stand der Technik dargestellte, polyklonale Antikörper werden gleichermaßen eingestellt.

Zum Nachweis von AK-IgG/POD-Konjugat wird ein Substratsystem oder eine Substratzubereitung verwandt, enthaltend Wasserstoffperoxid und Tetramethylbenzidin (TMB), welche aus zwei Stammlösungen hergestellt wird.

Stammlösung 1: TMB-Dihydrochlorid wird unter Rühren in einer Konzentration von 5 g/l, d. h. von 16 mmol/l in bidestilliertem Wasser gelöst und mit 5 normaler Salzsäure auf pH 1,5 eingestellt. Zu dieser Lösung wird Penicillin G unter Rühren in einer Endkonzentration von 200 mg/l, d. h. von 0,56 mmol/l zugesetzt.

Stammlösung 2: zu 900 ml bidestilliertem Wasser wird 1,4 ml Eisessig, 1,5 ml 1 normale NaOH und 250 mg, d. h. 3 mmol H₂O₂ als Harnstoff-Wasserstoffperoxid-Addukt zugesetzt. Nach vollständigem Lösen wird mit bidestilliertem Wasser auf 1 l aufgefüllt.

TMB-Substratzubereitung: Ein Volumenteil der Stammlösung 1 und 10 Volumenteile der Stammlösung 2 wird miteinander vermischt.

### Schritt 5

### Bestimmung von Antigen in einem ELISA mit den erfindungsgemäßen Peptiden

10 µl einer 0,01 mg/ml des gemäß Schritt 3 hergestellten fos-Peptid-Antikörperkonjugates enthaltenden Lösung in Kochsalz/Natriumcitrat-Puffer werden in die Näpfchen von Mikrotitrationsplatten die, wie beschrieben, mit Peptiden beschichtet werden, gegeben. Gegebenenfalls kann nach 10 - 30 min die Lösung abgesaugt und die Näpfchen gewaschen werden. 20 µl Serum oder Plasma, 10 µl Inkubationsmedium (BW, OSND) werden in die Näpfchen gegeben. Nach Inkubation über 30 Min. bei 37°C wird der Inhalt der Wells durch Absaugen entfernt und die Wells dreimal mit Waschpuffer enthaltend 1 g/l ^{R}Tween 20 in PBS gewaschen. Dann erfolgt die Zugabe von 100 µl endverdünntem Konjugat in die Wells. Nach einer Inkubation über 30 Min. bei 37°C wird der Inhalt der Wells durch Absaugen entfernt und wiederum dreimal gewaschen. Anschließend wird in jedes Näpfchen 100 µl TMB-Substratzubereitung gegeben, 30 Min. bei 20 - 22 °C inkubiert und die Inkubation durch Zugabe von 100 µl 1-normaler Schwefelsäure beendet. Die Extinktion der gefärbten Lösung wird bei einer Wellenlänge von 450 nm (E₄₅₀) gegen einen Leerwert von PBS gemessen.

### Beispiel 2

### Schritt 1

### Beschichtung von Mikrotitrationsplatten

150µl einer 5µg/ml jun- bzw. fos-Peptid enthaltenden Lösung in 50mM Carbonatpuffer werden jeweils in die Vertiefungen von Mikrotitrationsplatten der Fa. Nunc, Roskilde (Dänemark) gegeben. Die gefüllten Testplatten werden über Nacht bei Raumtemperatur belassen, anschließend die Lösungen in den Näpfchen abgesaugt und die Näpfchen dreimal mit 250µl einer phosphatgepufferten physiologischen Kochsalzlösung durch Füllen und Absaugen gewaschen. Die Testplatten läßt man hiernach über Kieselgel bei 20° C trocknen.

### Schritt 2

### Herstellung eines Maleimido-Antikörpers

Der zu konjugierende Antikörper (Konzentration 4mg/ml in PBS; 1 ml) wird mit Lithiumborat-Puffer (1ml) vermischt. Zu der resultierenden Lösung mit einem pH von 8,0 werden unter Rühren 0,017 ml einer 13mg/ml Lösung von GMBS (N-Gamma-Maleimidobutyryloxy-succinimid) in Dioxan (entsprechend einem 30fachen molaren Überschuß von GMBS gegenüber IgG) zugegeben. Nach einstündiger Inkubation bei Raumtemperatur wird der Reagenzüberschuß durch Gelfiltration an einer mit Phosphatpuffer pH 6,0 mit 5µM Titriplex I äquilibrierten Sephadex G-25-Säule entfernt.

### Schritt 3

### Kopplung des jun- bzw. fos-Leuzin-Reißverschlußpeptides an einen Maleimido-Antikörper

Der gemäß Schritt 2 hergestellte Maleimido-Antikörper (2mg in 2ml Phosphatpuffer pH 6,0 mit 5mM Titriplex I) wird mit einem Äquivalent jun- bzw. fos-Leuzin-Reißverschlußpeptid (15mg in 1ml PBS) 1 Stunde bei Raumtemperatur inkubiert. Dann wird über eine Sephadex G-25-Säule (Durchmesser 1cm; Höhe 15cm) mit 50mM Tris pH 7,4 gelfiltriert.

### Schritt 3 a)

### Kopplung eines funktionalisierten jun- bzw. fos-Leuzin-Reißverschlußpeptides an einen Maleimido-Antikörper

2,1mg jun- bzw. fos-Peptid werden in 0,21ml PBS aufgenommen und mit 9,5µl einer SAMSA-Stammlösung (S-Acetyl-Mercapto-Bernstein-säureanhydrid; FLUKA; 22,2mg/ml in Dioxan) versetzt. Nach 30 Minuten Reaktion bei Raumtemperatur wird die Lösung mit 60µl einer 1M NH₂OH in Wasser dotiert und weitere 15 Minuten bei Raumtemperatur inkubiert. Der Reaktionsansatz wird anschließend über eine Biogel P2-Säule mit Phosphatpuffer pH 6 mit 5µM Titriplex I entsalzt.

Die weitere Kopplungsprozedur an einen Maleimido-Antikörper erfolgt analog Schritt 3.

### Schritt 4

### Bestimmung von CEA in einem ELISA mit den erfindungsgemäßen Peptiden

100µl eines 0,01 mg/ml gemäß Schritt 2 hergestellten fos-Peptid-Anti-cEA-Antikörperkonjugates in Puffer (OSND, Behringwerke) werden in die Näpfchen von Mikrotitrationsplatten, die, wie beschrieben, mit jun-Peptid beschichtet wurden, gegeben. Gegebenenfalls kann nach 2 Stunden bei 37° C die Lösung abgesaugt und die Näpfchen mit Enzygnost-Waschpuffer (OSEW) gewaschen werden. 20µl Serum oder CEA-Standards und 100µl des endverdünnten Anti-CEA-Antikörpers (z.B. polyklonal, Kaninchen), der peroxidasemarkiert vorliegt, werden in die Näpfchen gegeben. Nach 2 Stunden Reaktionszeit bei 37° C wird der Inhalt der wells durch Absaugen entfernt und wiederum mit Waschpuffer gewaschen. Abschließend wird in jedes Näpfchen 100µl TMB-Substratzubereitung gegeben, 30 Minuten bei Raumtemperaturen inkubiert und die Inkubation durch Zugabe von 100µl 1-normaler Schwefelsäure beendet. Die Extinktion der gefärbten Lösung wird bei einer Wellenlänge von 450nm gegen einen Leerwert von PBS gemessen.

### Ausführungsbeispiel A: Standardkurve des CEA-ELISAs mit den erfindungsgemäßen Peptiden

**Tabelle 1**

| CEA-Konzentration (ng/ml) | 0 | 1 | 3 | 10 | 30 | 100 |
|---|---|---|---|---|---|---|
| Extinktion 450nm (mE) | 14 | 40 | 93 | 321 | 980 | 2471 |
| | 17 | 37 | 103 | 346 | 1067 | 2408 |
| Extinktionsmittelwert (mE) | 16 | 40 | 98 | 334 | 1024 | 2440 |

### Ausführungsbeispiel B:

### Korrelation des CEA-ELISAs mit den erfindungsgemäßen Peptiden im Vergleich mit anderen kommerziellen CEA-Tests

Die CEA-Bestimmungen wurden mit einem ELISA mit den erfindungsgemäßen Peptiden analog Ausführungsbeispiel A ermittelt, diesmal aber im inversen System mit einer fos-Peptid beschichteten Mikrotitrationsplatte und einem jun-Anti-CEA-Konjugat.

**Tabelle 2:**

| Übereinstimmung der CEA-Konzentrationen im Vergleich mit einem kommerziellen Test | | | | | | |
|---|---|---|---|---|---|---|
| CEA-Konzentrationen (ng/ml) in Normalseren | | | | | | |
| Enzygnost CEA micro (Behringwerke AG) | 1,49 | 1,32 | 1,05 | 1,05 | 1,26 | <1,0 |
| "jun/fos"-CEA ELISA | 1,22 | 1,94 | 1,5 | 1,88 | 1,74 | 1,3 |
| | | | | | | |

| CEA-Konzentrationen (ng/ml) in Tumorseren | | | | | | |
|---|---|---|---|---|---|---|
| Enzygnost CEA micro (Behringwerke AG) | 3,6 | 6,9 | 7,2 | 17,6 | 9,8 | 28,6 |
| "jun/fos"-CEA ELISA | 3,53 | 9,3 | 9,8 | 20,7 | 10,1 | 27,8 |
| Enzygnost CEA micro (Behringwerke AG) | 39,9 | 29,3 | 87,2 | 105,5 | 1538 | 5180 |
| "jun/fos"-CEA ELISA | 36,2 | 36,2 | 121,6 | 106,9 | 1276 | 5201 |

### Ergebnis:

Der direkte Vergleich mit einem kommerziellen CEA-Testkit ergab eine sehr gute Korrelierbarkeit der CEA-Konzentrationen (Korrelationskoeffizient r= 0,999).

### Schritt 5

### Bestimmung von AFP in einem ELISA unter Verwendung der erfindungsgemäßen Peptide

Je 100 µl eines gemäß Beispiel 2, Schritt 3 bzw. 3a hergestellten jun-Peptid-Anti-AFP-Antikörperkonjugates (Anti-AFP-Antikörper vom Schaf) werden in die Vertiefungen von Mikrotitrationsplatten, die gemäß Beispiel 2, Schritt 1 mit fos-Peptid beschichtet waren, pipettiert und 2 h bei 37°C inkubiert. Nach dreimaligem Waschen der Vertiefungen mit Waschpuffer (OSEW, Behringwerke) wurden je 20 µl Standard bzw. Probe und 100 µl eines Puffers (OSND, Behringwerke) mit Zusatz von Peroxidase-konjugierten Anti-AFP-Antikörpern vom Schaf eingefüllt. Die zweistündige Inkubation bei 37°C wird mit einem erneuten Waschschritt (s.o.) abgeschlossen und die Substrat/Chromogenreaktion gemäß Beispiel 2, Schritt 4 durchgeführt.

Entsprechend der gewählten Konzentration der Peroxidasekonjugierten Anti-AFP-Antikörper werden die jun-Peptid-Anti-AFP-Antikörper in einem Konzentrationsbereich von 25 bis 0.5 µg/ml eingesetzt, so daß das Meßsignal bei 450 nm für den Standard S5 (300 IU/ml) eine Mindestextinktion von 0.8 E beträgt.

### Ausführungsbeispiele jun/fos-AFP-Test:

### A) Spezifität der jun-fos-Interaktion I

Um die Spezifität der jun-fos-Interaktion zu überprüfen, wurden verschiedene Peptid-beschichtete bzw. unbeschichtete Mikrotitrationsplatten in Kombination mit Anti-AFP-Antikörpern, an die jun- oder fos-Peptid bzw. kein Peptid gekoppelt worden waren, analog zur oben beschriebenen Testdurchführung eingesetzt.

Ergebnis: Nur bei Verwendung der "fos-Festphase" in Kombination mit jun-Anti-AFP-Antikörpern konnte mit dem Standard S5 (300 IU AFP/ml) ein signifikant höheres Meßsignal im Vergleich zum Standard S0 (0 IU AFP/ml) erzielt werden (Tab. 3).

**Tabelle 3:**

| Spezifität der jun-fos-Interaktion | | |
|---|---|---|
| Meßsignal (Extinktion) | | |
| Standard S0 (0 IU AFP/ml) | Standard S5 (300 IU AFP/ml) | |
| a) Mikrotitrationsplatte unbeschichtet: | | |
| jun-Anti-AFP-Antikörper: | 0.007 | 0.026 |
| fos-Anti-AFP-Antikörper: | 0.011 | 0.017 |
| Anti-AFP-Antikörper: | 0.009 | 0.017 |

| b) jun-beschichtete Mikrotitrationsplatte: | | |
|---|---|---|
| jun-Anti-AFP-Antikörper: | 0.008 | 0.061 |

| c) fos-beschichtete Mikrotitrationsplatte: | | |
|---|---|---|
| jun-Anti-AFP-Antikörper: | 0.006 | 3.250 |
| fos-Anti-AFP-Antikörper: | 0.009 | 0.012 |
| Anti-AFP-Antikörper: | 0.009 | 0.017 |
| kein Anti-AFP-Antikörper: | 0.010 | 0.011 |

### B) Spezifität der jun-fos-Interaktion II

Dem weiter oben beschriebenen jun/fos-AFP-Test wurde ein einstündiger Inkubationsschritt bei 37°C mit je 100 µl einer jun- bzw. fos-Peptid-haltigen Pufferlösung (Puffer = OSND) pro Vertiefung vorgeschaltet. Nach 3-maligem Waschen erfolgte die weitere Assaydurchführung gemäß obiger Vorschrift.

Ergebnis: Nur durch Zugabe von freiem jun-Peptid konnte die Anbindung von jun-Anti-AFP-Antikörper an die fos-beschichtete Festphase inhibiert werden (Tab. 4)

**Tab.4**

| Inhibition der jun-Anti-AFP-Antikörper-Bindung an die fos-beschichtete Mikrotitrationsplatte durch Zugabe freien Peptids | | |
|---|---|---|
| freies Peptid (µg/ml) | Meßsignal (Extinktion 450 nm) | |
| | Standard S0 (0 IU AFP/ml) | Standard S5 (300 IU AFP/ml) |
| jun 0 | 0.010 | 3.590 |
| 5 | 0.015 | 3.486 |
| 50 | 0.011 | 2.857 |
| 500 | 0.013 | 1.371 |
| | | |
| fos 0 | 0.007 | 3.599 |
| 5 | 0.010 | 3.416 |
| 50 | 0.011 | 3.572 |
| 500 | 0.015 | 3.694 |

### C) Standardkurve jun/fos-AFP-Test:

**Tab.5:**

| Standardkurve des jun/fos-AFP-Testes | | | | | | |
|---|---|---|---|---|---|---|
| AFP-Konzentration (IU/ml) | 0 | 3 | 10 | 30 | 100 | 300 |
| Extinktion 450 nm | 0.009 | 0.021 | 0.101 | 0.396 | 0.631 | 0.883 |
| | 0.009 | 0.024 | 0.071 | 0.296 | 0.699 | 0.916 |
| Extinktionsmittelwert | 0.009 | 0.023 | 0.086 | 0.346 | 0.665 | 0.900 |

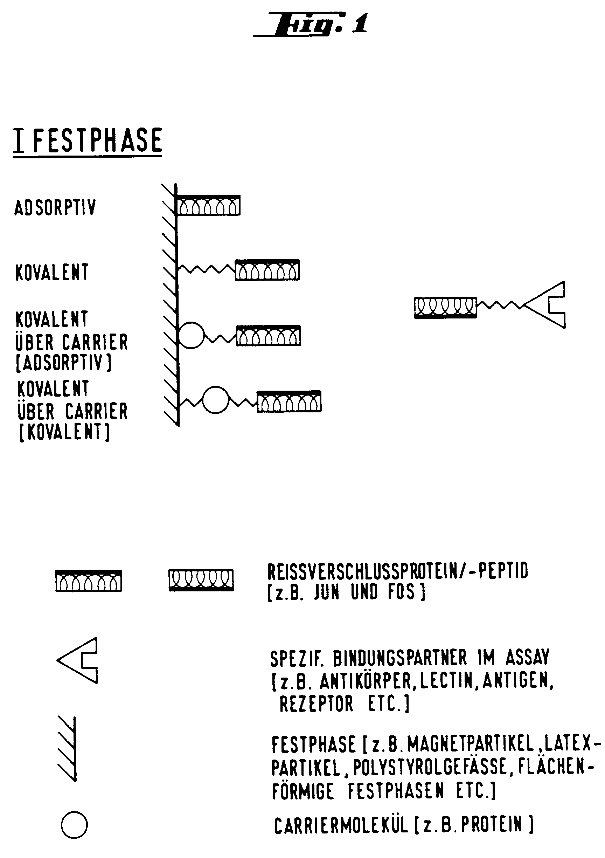

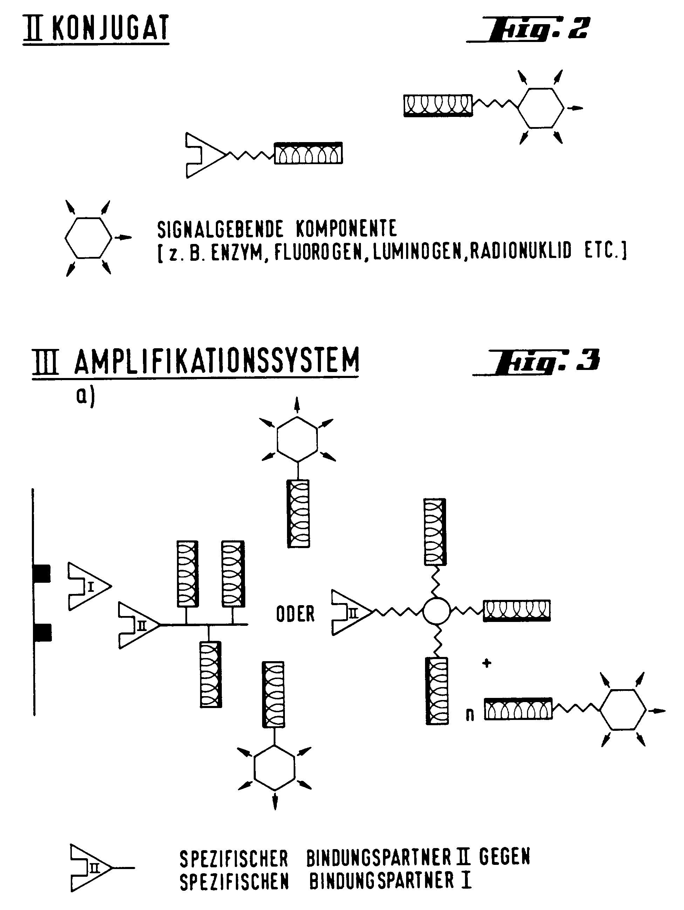

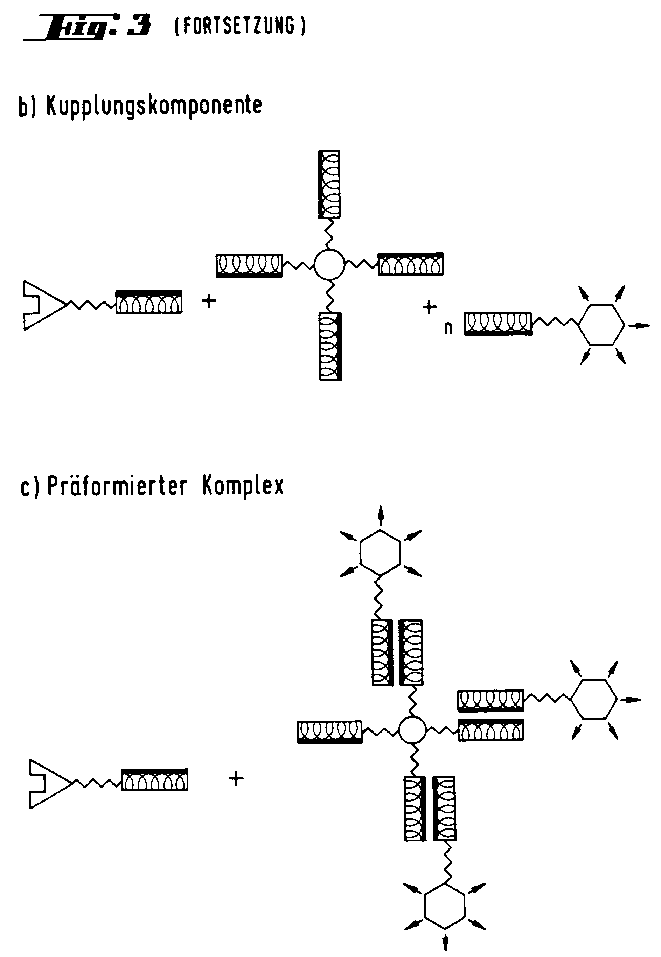

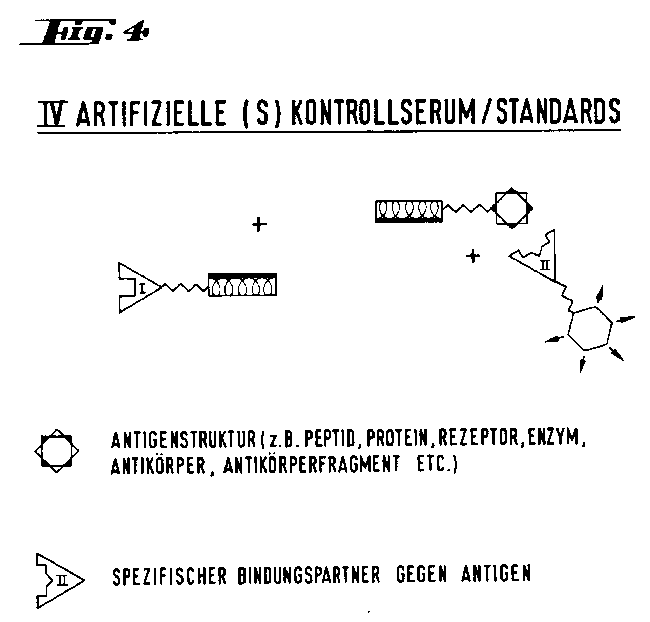

## Patentansprüche

1. Verwendung von Peptidpaaren mit extrem hoher spezifischer Affinität zueinander im Bereich der in vitro Diagnostik, dadurch gekennzeichnet, daß diese Peptide "Leucin-Reißverschlußpeptide" sind.

2. Verwendung gemäß Anspruch 1, dadurch gekennzeichnet, daß in einem Festphasenimmunoassay ein Peptid des "Leucin-Reißverschluß" Peptidpaares adsorptiv oder kovalent, direkt oder über einen Spacer an die Festphase gebunden ist und das zweite Peptid des "Leucin-Reißverschluß" Peptidpaares an den zur Immobilisierung des nachzuweisenden Analyten dienenden spezifischen Bindungspartner für den nachzuweisenden Analyten gebunden ist.

3. Verwendung gemäß Anspruch 1, dadurch gekennzeichnet, daß in einem Immunoassay der zum Nachweis dienende spezifische Bindungspartner an ein Peptid des "Leucin-Reißverschluß" Peptidpaares gebunden ist und das zweite Peptid dieses Paares an eine signalgebende Komponente gebunden ist.

4. Verwendung gemäß Anspruch 3, dadurch gekennzeichnet, daß an den spezifischen Bindungspartner direkt oder über ein Carriermolekül mehr als ein Molekül des zweiten Peptids des "Leucin-Reißverschluß" Peptidpaares gebunden ist.

5. Verwendung gemäß Anspruch 1, dadurch gekennzeichnet, daß ein spezifischer Bindungspartner für ein Antigen über ein "Leucin-Reißverschluß" Peptidpaar mit einer Antigenstruktur verknüpft ist, die von einem zweiten spezifischen Bindungspartner des Analyten als spezifischer Bindungspartner erkannt wird.

6. Heterogenes Immunchemisches Verfahren zur Bestimmung eines Analyten, dadurch gekennzeichnet, daß der an die Festphase gebundene spezifische Bindungspartner über ein "Leucin-Reißverschluß" Peptidpaar an die Festphase gebunden ist.

7. Verfahren gemäß Anspruch 6, dadurch gekennzeichnet, daß die Festphase eine Mikrotitrationsplatte oder ein magnetisch anziehbarer Partikel ist.

8. Heterogenes immunchemisches in vitro Verfahren zur Bestimmung eines Analyten, dadurch gekennzeichnet, daß der zum Nachweis dienende spezifische Bindungspartner an ein Peptid des "Leucin-Reißverschluß" Peptidpaares gebunden ist und das zweite Peptid dieses Paares eine signalgebende Komponente trägt.

9. Verfahren gemäß Anspruch 8, dadurch gekennzeichnet, daß an den spezifischen Bindungspartner direkt oder über einen Carriermolekül mehr als ein Molekül des zweiten Peptids des "Leucin-Reißverschluß" Peptidpaares gebunden ist.

10. Verwendung gemäß Anspruch 1, dadurch gekennzeichnet, daß in einem Immunoassay an dem zum Nachweis dienenden spezifischen Bindungspartner direkt oder über ein Carriermolekül mehr als ein Peptid des "Leucin-Reißverschluß" Peptidpaares gebunden ist und das zweite Peptid dieses Paares eine signalgebende Komponente trägt.

11. Universelle Festphase zur Verwendung in heterogenen immunchemischen Verfahren zur Bestimmung eines Analyten, dadurch gekennzeichnet, daß ein Peptid des "Leucin-Reißverschluß" Peptidpaares absorptiv oder kovalent, direkt oder über einen Spacer an die Festphase gebunden ist.

12. Universelles Konjugat zur Verwendung in immunchemischen Verfahren, dadurch gekennzeichnet, daß an den zum Nachweis dienenden spezifischen Bindungspartner mindestens ein Peptid des "Leucin-Reißverschluß" Peptidpaares gebunden ist und das zweite Peptid dieses Paares an eine signalgebende Komponente gebunden ist.

## Claims

1. The use of pairs of peptides with extremely high specific affinity for one another in the area of in vitro diagnosis, wherein these peptides are "leucine zipper peptides".

2. The use as claimed in claim 1, wherein in a solidphase immunoassay one peptide of the pair of "leucine zipper" peptides is attached by adsorption or covalently, directly or via a spacer, to the solid phase, and the second peptide of the pair of "leucine zipper" peptides is bound to the specific binding partner, which serves for immobilization of the analyte to be detected, for the analyte to be detected.

3. The use as claimed in claim 1, wherein in an immunoassay the specific binding partner serving for detection is bound to one peptide of the pair of "leucine zipper" peptides, and the second peptide of this pair is bound to a signal-emitting component.

4. The use as claimed in claim 3, wherein more than one molecule of the second peptide of the pair of "leucine zipper" peptides is bound to the specific binding partner, directly or via a carrier molecule.

5. The use as claimed in claim 1, wherein a specific binding partner for an antigen is linked via a pair of "leucine zipper" peptides to an antigenic structure which is recognized as specific binding partner by a second specific binding partner of the analyte.

6. A heterogeneous immunochemical method for the determination of an analyte, wherein the specific binding partner bound to the solid phase is bound to the solid phase via a pair of "leucine zipper" peptides.

7. The method as claimed in claim 6, wherein the solid phase is a microtiter plate or a magnetically attractable particle.

8. A heterogeneous immunochemical in vitro method for the determination of an analyte, wherein the specific binding partner serving for detection is bound to one peptide of the pair of "leucine zipper" peptides, and the second peptide of this pair carries a signal-emitting component.

9. The method as claimed in claim 8, wherein more than one molecule of the second peptide of the pair of "leucine zipper" peptides is bound to the specific binding partner, directly or via a carrier molecule.

10. The use as claimed in claim 1, wherein in an immunoassay more than one peptide of the pair of "leucine zipper" peptides is bound to the specific binding partner serving for detection, directly or via a carrier molecule, and the second peptide of this pair carries a signal-emitting component.

11. A universal solid phase for use in heterogeneous immunochemical methods for the determination of an analyte, wherein one peptide of the pair of "leucine zipper" peptides is attached by absorption or covalently, directly or via a spacer, to the solid phase.

12. A universal conjugate for use in immunochemical methods, wherein at least one peptide of the pair of "leucine zipper" peptides is bound to the specific binding partner serving for detection, and the second peptide of this pair is bound to a signal-emitting component.

## Revendications

1. Utilisation de paires de peptides présentant une très forte affinité spécifique l'un pour l'autre dans le domaine du diagnostic in vitro, caractérisée en ce que ces peptides sont des peptides à séquence à pontage leucine.

2. Utilisation selon la revendication 1, caractérisée en ce que dans un immunoessai en phase solide, un peptide de la paire de peptides à séquence à pontage leucine est lié par adsorption ou de façon covalente, directement ou par l'intermédiaire d'un espaceur à la phase solide et en ce que le second peptide de la paire de peptides à séquence à pontage leucine est lié au partenaire de liaison spécifique de l'analyte à détecter utilisé pour l'immobilisation de l'analyte à détecter.

3. Utilisation selon la revendication 1, caractérisée en ce que dans un immunoessai, le partenaire de liaison spécifique utilisé pour la détection est lié à un peptide de la paire de peptides à séquence à pontage leucine et le second peptide de cette paire est lié à un composant capable d'émettre un signal.

4. Utilisation selon la revendication 3, caractérisée en ce plus d'une molécule du second peptide de la paire de peptides à séquence à pontage leucine est liée au partenaire de liaison spécifique directement ou par l'intermédiaire d'une molécule porteuse.

5. Utilisation selon la revendication 1, caractérisée en ce qu'un partenaire de liaison spécifique d'un antigène est lié par l'intermédiaire d'une paire de peptides à séquence à pontage leucine à une structure antigénique, laquelle est reconnue par un second partenaire de liaison spécifique de l'analyte en tant que partenaire de liaison spécifique.

6. Procédé immunochimique hétérogène pour la détermination d'un analyte, caractérisé en ce que le partenaire de liaison spécifique lié à la phase solide est lié à la phase solide par l'intermédiaire d'une paire de peptides à séquence à pontage leucine.

7. Procédé selon la revendication 6, caractérisé en ce que la phase solide est une plaque pour microtitration ou une particule pouvant être attirée magnétiquement.

8. Procédé immunochimique hétérogène in vitro pour la détermination d'analytes, caractérisé en ce que le partenaire de liaison spécifique utilisé pour la détermination est lié à un peptide de la paire de peptides à séquence à pontage leucine et en ce que le second peptide de cette paire porte un composant capable d'émettre un signal.

9. Procédé selon la revendication 8, caractérisé en ce que plus d'une molécule du second peptide de la paire de peptides à séquence à pontage leucine est liée directement ou par l'intermédiaire d'une molécule porteuse au partenaire de liaison spécifique.

10. Utilisation selon la revendication 1, caractérisée en ce que dans un immunoessai, plus d'un peptide de la paire de peptides à séquence à pontage leucine est lié directement ou par l'intermédiaire d'une molécule porteuse, au partenaire de liaison spécifique utilisé pour la détermination et en ce que le second peptide de cette paire porte un composant capable d'émettre un signal.

11. Phase solide universelle pour l'utilisation dans un procédé immunochimique hétérogène pour détermination d'un analyte, caractérisée en ce qu'un peptide de la paire de peptides à séquence à pontage leucine est lié à une phase solide par absorption ou par liaison covalente directement ou par l'intermédiaire d'un espaceur.

12. Conjugué universel pour l'utilisation dans un procédé immunochimique, caractérisé en ce qu'au moins un peptide de la paire de peptides à séquence à pontage leucine est lié au partenaire de liaison spécifique utilisé pour la détermination et en ce que le second peptide de cette paire est lié à un composant capable d'émettre un signal.
